# EUROPEAN PATENT SPECIFICATION

(11) **EP 1 140 333 B1**
(45) Date of publication and mention of the grant of the patent: **23.07.2003**
(21) Application number: 99958024.4
(22) Date of filing: 09.11.1999
(51) Int. Cl.: B01D 69/02, B01D 67/00, A61L 2/23, A61L 15/46

(54) **MICROPOROUS FLAT, CAPILLARY OR TUBULAR MEMBRANE AND METHOD OF MANUFACTURING THE SAME**
MIKROPORÖSE FLACH-, KAPILLAR- ODER ROHRMEMBRAN UND VERFAHREN ZU IHRER HERSTELLUNG
MEMBRANE MICROPOREUSE PLATE, CAPILLAIRE OU TUBULAIRE ET PROCEDE DE FABRICATION CORRESPONDANT

(30) Priority: 16.11.1998 NL 1010559
(43) Date of publication of application: 10.10.2001
(73) Proprietor: Prime Membrane Technologies N.V., 2400 Mol (BE)
(72) Inventor: ADRIANSENS, Walter, Ludovicus, Carolus, B-2400 Mol (BE); GENNE, Inge, Josfien, Marino, B-3500 Hasselt (BE); SCHARSTUHL, Johan, Jan, NL-7495 TC Ambt Delden (NL)
(74) Representative: Kupecz, Arpad
(86) International application number: EP9908695
(87) International publication number: WO00029101

(56) References cited:
- GB-A- 1 521 171
- US-A- 5 006 267
- US-A- 5 102 547
- US-A- 5 670 646
- PATENT ABSTRACTS OF JAPAN vol. 1996, no. 10, 31 October 1996 (1996-10-31) & JP 08 157637 A (UNITIKA LTD), 18 June 1996 (1996-06-18)

## Description

The present invention relates to a microporous flat, capillary or tubular membrane and method of manufacturing the same.

Such a membrane and method of its manufacture are known from EP 0 241 995 B1 and EP 0 624 283 B1. The known membranes comprise polysulphones and zirconium oxide, with polysulphone being responsible for the mechanical structure of the membrane, while the zirconium oxide provides the permeability and the hydrophilic nature.

There is an increasing incidence of infectious diseases caused by bacteria and viruses, while at the same time the effectiveness of antibiotics is declining due to emerging new kinds of microorganisms which have become resistant to the known antibiotics.

In order to eliminate such microorganisms, chemical means are used, which chemical means, however, have the disadvantage that they are very toxic and have very undesirable side-effects which have been shown to be detrimental to health.

All over the world research is directed toward killing or filtering out microorganisms, without side-effects, before they become active in humans.

S.D. Worley et al in Trends in Polymers Science, Vol. 4, No. 11, November 1996 have described biocidal polymers in the form of an amorphous solid material (granulate with a particle size between 20 µm and 300 µm) which is insoluble in water.

It has been shown that such biocidal polymers are capable of killing harmful microorganisms present in air and water.

It has also been shown that such biocidal polymers cannot simply be used efficiently for the sterilization of water and air on an acceptably workable level.

Moreover, membranes have been developed in particular for the sterilization of water, which are better capable of retaining very small microorganisms. However, the draw-back of these membranes is, that due to their small pores they have an inadequate flow velocity. Another draw-back of such membranes is the occurrence of bio-fouling, so that the pores become clogged.

US-A-5 670 646 discloses monomeric and polymeric cyclic amine and halamine compounds as such.

US-A-5 006 267 relates to biocidal fluid filters.

US-A-5 102 547 discloses anti-fouling semi-permeable membrane system.

The object of the present invention is to efficiently use the above-described biocidal polymers for the purification of water and air.

To this end the present invention provides a microporous membrane, flat, capillary or tubular, wherein the matrix of the membrane comprises a water-insoluble biocidal compound which is a N-halamine polymer, and preferably a polystyrene hydantoin or a polystyrene triazinedione such as described by S.D. Worley et al.

Surprisingly, such a biocidal polymer-comprising microporous membrane is particularly suitable for the sterilization of water despite the large pores that are necessary for good flow while in addition, bio-fouling of the membrane is reduced.

If such a membrane is hydrophobic, it has been shown to also be suitable for the disinfection of air.

The inclusion of a biocidal polymer in the matrix of the membrane appears to effectuate a complete inactivation of the bacteria and viruses, by way of supplementing the membrane's filtration properties.

A particularly suitable membrane according to the invention is one comprising a polystyrene hydantoin with the formula 1 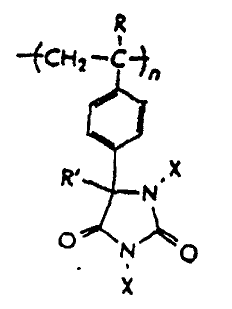 wherein
X = Cl, Br, J
R = H, CH₃
R' = an alkyl group
n = 10 - 50.10³
or a polystyrene triazinedione with the formula 2 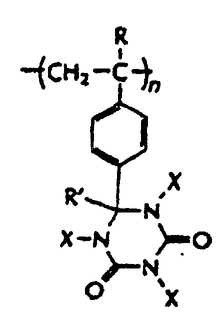 wherein
X = Cl, Br, J
R = H, CH₃
R' = an alkyl group
n = 10 - 50.10³

Outstandingly effective membranes are membranes according to the invention, which as biocidal compound comprise poly-1,3-dichloro(dibromo, di-iodo)-5-methyl-5-(4'-vinylphenyl)hydantoin. In general, a good and adequate biocidal effect is obtained when the membrane comprises 0.5-25% by weight of poly-1,3-dichloro(dibromo, di-iodo)-5-methyl-5-(4'-vinylphenyl)hydantoin.

When using the membrane according to the invention for water sterilization purposes, the membrane is effective when it comprises 0.5-12% by weight of poly-1,3-dichloro(dibromo, di-iodo)-5-methyl-5-(4'-vinylphenyl)-hydantoin, whereas if one wishes to use the membrane according to the invention for the disinfection of air, it is advantageous if the membrane comprises 12-25% by weight of poly-1,3-dichloro(dibromo,di-iodo)-5-methyl-5-(4'-vinylphenyl)hydantoin.

The invention further relates to a membrane, suitable for use as sterile disinfecting dressing for wounds, which membrane comprises 0.5-12% by weight of poly-1,3-di-iodo-5-methyl-5-(4'-vinylphenyl)hydantoin.

The invention further relates to a method of manufacturing a hydrophilic microporous membrane or of manufacturing a hydrophobic microporous membrane.

The method according to the invention for the manufacture of a hydrophilic microporous membrane is characterized in that a membrane for water sterilisation or for wound dressing is manufactured by thoroughly mixing an organic solvent, a hydrophilic substance, a substance that does or does not form pores, a hydrophilic polymer and a water insoluble biocidal polymer with the formula 1 or 2, wherein X, R, R' and n have the meanings defined in claim 4, after which the mixture is cast onto a support, coagulated in a water bath, optionally followed by treatment with an anorganic acid, rinsing and drying.

Preferably as organic solvent 41.4% by weight N-methylpyrrolidone, 5.6% by weight polysulphone, 17% by weight zirconium oxide, 34% by weight calcium carbonate, 0,5% by weight polyvinylpyrrolidone and 1.5% by weight poly-1,3-dichloro(dibromo)-5-methyl-5-(4'-vinylphenyl) hydantoin are thoroughly mixed together, after which the mixture obtained is cast onto a support, forming a film which is subsequently coagulated in a bath of N-methylpyrrolidone and water at room temperature, followed by treatment with a hydrochloric acid solution to dissolve calcium carbonate, after which the obtained membrane is rinsed with water at approx. 100°C and dried.

Apart from N-methylpyrrolidone, which is preferred, dimethylformamide, dimethylacetamide, chloroform, tetrahydrofuran, etc. may be used as solvent.

In stead of zirconium oxide, aluminium oxide and titanium oxide may be used successfully.

Apart from CaCO₃, (NH₄)₂CO₃ or other suitable organic or anorganic pore formers may suitably be used for the formation of pores.

According to the invention, a hydrophobic membrane for the disinfection of air is manufactured by thoroughly mixing a solvent, polysulphone, a pore-forming compound and a poly-1,3-dichloro(dibromo)-5-methyl-5-(4'-vinylphenyl)hydantoin, after which the obtained mixture is cast onto a support forming a film, which is then washed twice, after which the pore-forming medium is dissolved in an acid, followed by rinsing the membrane and immersing the membrane in warm water.

A particularly favourable hydrophobic membrane is obtained according to the invention by thoroughly mixing 40% by weight N-methylpyrrolidone, 7.0% by weight polysulphone, 34% by weight calcium carbonate and 19.0% by weight poly-1,3-dichloro(dibromo)-5-methyl-5-(4'-vinylphenyl)hydantoin, casting the obtained mixture onto a support and coagulating in a bath of 74.5% by weight N-methylpyrrolidone and 25.5% by weight water at room temperature, thereby producing a film, which is subsequently immersed in the same bath, after which the CaCo₃ in the film is dissolved by treatment with hydrochloric acid, followed by rinsing the film with water and immersing it in water of approx. 100°C for one hour.

The invention will now be further elucidated with reference to the following examples.

### Example 1

Starting from the method described in EP 0 624 283 B1, wherein the matrix comprises poly-1,3-dichloro(dibromo)-5-methyl-5-(4'-vinylphenyl)hydantoin as biocidal compound, a very effectively biocidal membrane was obtained, which membrane was shown to be particularly suitable for the sterilisation of water.

In this example the compounds below were thoroughly mixed by high speed stirring:

| | |
|---|---|
| N-methylpyrrolidone (NMP) | 41.4% by weight |
| Polysulphone (Psf) | 5.6% by weight |
| Zirconium oxide (ZrO₂) | 17.0% by weight |
| Calcium carbonate (CaCo₃) | 34.0% by weight |
| Polyvinylpyrrolidone (PVP) | 0.5% by weight |
| poly-1,3-dichloro(dibromo) -5-methyl-5-(4'-vinylphenyl) hydantoin | 1.5% by weight. |

After a homogenous mixture was obtained it was cast onto a support at a film thickness of 200 µm, after which the film obtained was subjected to coagulation in a bath of 74.5% by weight NMP and 25.5% by weight water at room temperature. The membrane was subsequently immersed in a second bath of the same composition at room temperature for at least 20 minutes.

Then the pore structure was realized by dissolving calcium carbonate in a 2 molar hydrochloric acid solution. After that the membrane was rinsed and immersed in water of 100°C for 1 hour and finally dried.

By this method a flat hydrophilic microfiltration membrane was obtained having a pore size of approx. 3 µm and a 6% final concentration of poly-1,3-dichloro(dibromo)-5-methyl-5-(4'-vinylphenyl) hydantoin in the membrane.

In a similar manner membranes were obtained having a final concentration of poly-1,3-dichloro(dibromo)-5-methyl-5-(4' vinylphenyl) hydantoin of 2% and 12% in the membrane, with a pore size of approx. 3 µm.

The microporous membranes comprising 2%, 6% and 12% poly-1,3-dichloro(dibromo)-5-methyl-5-(4'-vinylphenyl) hydantoin, manufactured according to example 1, were examined with respect to their water sterilising activity by placing them in a sterile vessel containing 50 ml water and a certain concentration of Pseudomonas diminuta. After a particular period of time a 0.5 ml sample was taken, incubated and measured at approx. 60-second intervals, in order to determine the membrane's bacteria-killing activity.

It was shown that after 1 hour a 2% membrane still exhibited bacterial activity.

Using a membrane with 6% by weight of the N-halamine, the bacteria were shown to be dead in less than 1 hour, whereas when using a membrane with 12% by weight of N-halamine, all bacteria appeared to have been killed within 25 minutes.

Subsequently the membranes were attached to the end of a tube, through which water was conducted that was polluted with 3.5 x 10⁴ CFU Pseudomonas diminuta per 30 ml. P-diminuta is a bacterium having a size of 0.1 - 0.3 µm, which easily passes a membrane having an average pore size of approx. 3 µm. While the polluted suspension was conducted through the membrane, samples were taken at regular intervals of between 1 and 35 minutes. It was shown that membranes comprising 2% by weight of N-halamine reduce bacterial activity in every permeate sample by approx. 85%. No bacterial activity was registered in the permeate samples of the remaining membranes, comprising 6% and 12% respectively of the N-halamine.

The flow velocity of the polluted water through the membranes with a surface of approx. 38 cm² corresponded with 1 litre in 15 minutes.

Subsequently a comparative experiment was carried out in which the biocidal polymer poly-1,3-dichloro (dibromo)-5-methyl-5-(4' vinylphenyl) hydantoin as such was used.

A 10 mm thick layer of poly-1,3-dichloro(dibromo)-5-methyl-5-(4'-vinylphenyl)hydantoin was transferred onto a fine-mesh support in a tube having the same diameter, while the poly-1,3-dichloro(dibromo)-5-methyl-5-(4'-vinylphenyl) hydantoin was covered with the same kind of fine-mesh material. Similar bacteria-polluted water as mentioned above was introduced into the tube. The poly-1,3-dichloro(dibromo)-5-methyl-5-(4'-vinylphenyl)hydantoin had become a muddy paste, through which the water passed only drop by drop. The permeate samples from the middle of the tube showed no bacterial activity. Other samples from the walls of the tube did show bacterial activity, due to the fact that the walls were not completely sealed.

It will be clear, that when poly-1,3-dichloro(dibromo)-5-methyl-5-(4'-vinylphenyl)hydantoin is used as such, it does not offer a practicable application for the sterilisation of water. To obtain an effective practical system the N-halamine (polystyrene hydantoin) has to be incorporated in a mechanical structure such as in accordance with the invention, in a membrane matrix, thereby obtaining an efficient bacteria-killing activity at a good flow velocity.

Surprisingly it was shown that the membranes comprising the poly-1,3-dichloro(dibromo)-5-methyl-5-(4' vinylphenyl) hydantoin, also possessed good virus-killing activity.

It will be obvious that the microporous membrane according to the invention comprising the N-halamine (polystyrene hydantoin), may also be used in the form of a capillary or tubular membrane.

### Example 2

According to another embodiment, a membrane was prepared from

| | |
|---|---|
| N-Methylpyrrolidone (NMP) | 17.0% by weight |
| Polyethersulphone | 77.4% by weight |
| Poly-1,3-dichloro(dibromo)-5-methyl-5-(4'-vinylphenyl) hydantoin | 5.6% by weight. |

After thorough mixing the mixture was spun in a standard spinning apparatus, after which the product obtained was allowed to coagulate in water at approx. 60°C. The capillary ultra filtration membrane obtained comprised a final concentration of poly-1,3-dichloro(dibromo)-5-methyl-5-(4'-vinylphenyl)hydantoin of 6.7% at a cut-off of 150.000 Daltons and a flux of 500 l/m²/hour/bar.

A bundle of capillary membranes was made, using the capillary membranes from Example 2, and a bundle of capillary membranes was made that did not comprise poly-1,3-dichloro(dibromo)-5-methyl-5-(4' vinylphenyl) hydantoin. These bundles were placed into polluted water for 48 hours. The bio-fouling in the capillary membranes from Example 2 was shown to be reduced by a factor 4 compared with the capillary membranes without poly-1,3-dichloro(dibromo)-5-methyl-5- (4' vinylphenyl) hydantoin. This was established after 48 hours.

### Example 3

The following compounds were thoroughly mixed and subsequently cast onto a support, obtaining a film of 200 µm thickness.

| | |
|---|---|
| N-methylpyrrolidone | 61.5% by weight |
| Polyethersulphone | 20.0% by weight |
| 2 Metoxymethanol | 15.7% by weight |
| PEG 4000 | 0.9% by weight |
| Poly-1,3-dichloro(dibromo) -5-methyl-5-(4'-vinylphenyl) hydantoin | 1.9% by weight |

The film was allowed to coagulate in a water bath at approx. 60°C.

By this method a flat hydrophilic microfiltration membrane was obtained comprising 8.3% by weight of poly-1,3-dichloro(dibromo)-5-methyl-5-(4'-vinylphenyl) hydantoin and having a pore size of approx. 0.2 µm. The flux was 5000 l/m²/hour/bar.

A flat membrane according to Example 3 was produced, and a flat membrane without poly-1,3-dichloro(dibromo)-5-methyl-5-(4'-vinylphenyl) hydantoin, and placed into polluted water for 48 hours. Subsequently the bio-fouling in both types of membranes was examined, showing that the bio-fouling in the membranes from Example 3 was reduced by a factor 2 compared with the membranes without poly-1,3-dichloro(dibromo)-5-methyl-5-(4'-vinylphenyl)hydantoin. This was established after 48 hours.

Subsequently the membranes were attached to the end of a tube and water was passed through containing 1 x 10⁷/L Rotavirus.

While the water was passed through the membranes, samples were taken at regular intervals. The membrane from Example 3 was shown to be able to kill the Rotavirus in the permeate for 99.99%, while the membrane without the poly-1,3-dichloro(dibromo)-5-methyl-5-(4'-vinylphenyl) hydantoin only showed a reduction of 62%, which was probably due to the membranes' physical retention of the viruses.

It will be obvious that in stead of a flat microporous membrane it is also possible to produce a capillary or tubular membrane.

### Example 4

This example describes the composition and preparation of a flat flexible microfiltration membrane which, through the addition of poly-1,3-di-iodo-5-methyl-5-(4'-vinylphenyl) hydantoin can be used as a disinfecting wound dressing.

| | |
|---|---|
| N-methylpyrrolidone | 61.0% by weight |
| Ethanol | 25.0% by weight |
| Polyurethane (PU) | 8.0% by weight |
| Polyvinylpyrrolidone (PVP) | 4.0% by weight |
| Poly-1,3-di-iodo-5-methyl-5-(4'-vinylphenyl) hydantoin | 1.0% by weight. |

The N-methylpyrrolidone is added to the ethanol and subsequently stirred for 30 minutes, after which the mixture is subjected to vigorous mixing. The polyvinylpyrrolidone was then added gradually, while the mixer was stirring at 3000 rotations per minute. After all the polyvinylpyrrolidone was added mixing was continued for another 5 minutes.

After 1 hour of mixing, the polyurethane and the poly-1,2-di-iodo-5-methyl-5- (4'-vinylphenyl)hydantoin was added while stirring vigorously.

After degassing for 24 hours and subsequent filtration, the polymer solution is ready for use.

The polymer solution was cast onto a support in a film of approx. 200 µm thickness, after which the thus obtained film was allowed to coagulate in a water bath at 60°C.

The result was a hydrophilic flat microfiltration membrane, comprising 7.7% of the poly-1,3-di-iodo-5-methyl-5-(4'-vinylphenyl)hydantoin having a pore size of 0.2 µm. The membrane was flexible and foldable, making it particularly suitable for use as disinfecting wound dressing.

### Example 5

In this example a hydrophobic membrane is manufactured for the disinfection of air.

The following components were thoroughly mixed:

| | |
|---|---|
| N-methylpyrrolidone (NMP) | 40.0% by weight |
| Polysulphone (Psf) | 7.0% by weight |
| Calcium carbonate (CaCO₃ | 34.0% by weight |
| Poly-1,3-dichloro(dibromo) -5-methyl-5-(4'-vinylphenyl) hydantoin | 19.9% by weight. |

The mixture obtained was cast onto a support in a film having a thickness of 400 µm, after which the film was allowed to coagulate in a bath of 74.5% by weight NMP and 25.5% by weight water at room temperature. The membrane was subsequently immersed in a second bath of the same composition and temperature for at least 20 minutes.

Subsequently the pore structure was realized by dissolving the CaCO₃ in a 2 molar hydrochloric acid solution. Then the membrane was rinsed and immersed in water of approx. 100°C for approx. 1 hour. Finally the membrane was dried.

By this method a flat hydrophobic membrane was obtained having a pore size of approx. 1 µm and a final concentration of 73% of the poly-1,3-dichloro-5-methyl-5-(4'-vinylphenyl)hydantoin in the membrane.

After some time the membranes have to be reactivated as the chlorine- (bromine-, iodine-) atoms become extracted from the poly-1,3-dichloro(dibromo,di-iodo) -5-methyl-5-(4'-vinylphenyl) hydantoin after some time.

To this end the membranes are treated with a chlorinated solution. Obviously, if bromo- or iodo-comprising N-halamines are used, one uses a bromine- or iodine-comprising solution.

Quite conceivably a membrane with a cellulose structure in which a biocidal compound is comprised may also have a satisfactory biocidal effect.

It goes without saying that the invention is not limited to the above-mentioned examples.

## Claims

1. A microporous flat or capillary or tubular membrane, comprising a water-insoluble biocidal compound, **characterized in that** the matrix of the membrane comprises as biocidal compound a N-halamine polymer, and wherein the membrane comprises 0.5-25% by weight of the biocidal compound.

2. A membrane according to claim 1, **characterized in that** the N-halamine polymer is a polystyrene hydantoin with the formula 1 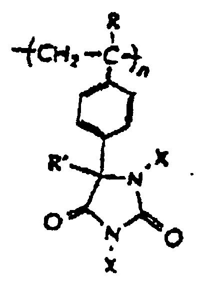 wherein
X = Cl, Br, J
R = H, CH₃
R' = an alkyl group
n = 10 - 50.10³
or a polystyrene triazinedione with the formula 2 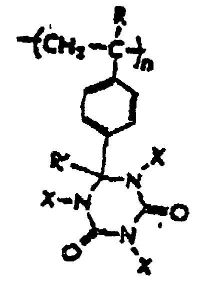 wherein
X = Cl, Br, J
R = H, CH₃
R' = an alkyl group
n = 10 - 50.10³

3. A membrane according to claims 1 or 2, **characterized in that** the membrane as biocidal compound comprises poly-1,3-dichloro(dibromo, di-iodo)-5-methyl-5-(4'-vinylphenyl)hydantoin.

4. A membrane according to claim 3, suitable for water sterilisation,
**characterized in that** the membrane comprises 0.5-12% by weight of poly-1,3-dichloro(dibromo)-5-methyl-5-(4'-vinylphenyl)hydantoin.

5. A membrane according to claim 3, suitable for air filtration, **characterized in that** the membrane comprises 12-25% by weight of poly-1,3-dichloro(dibromo)-5-methyl-5-(4'-vinylphenyl)hydantoin.

6. A membrane according to claim 3, suitable for sterile wound dressing,
**characterized in that** the membrane comprises 0.5-12% by weight of poly-1,3-di-iodo-5-methyl-5-(4'-vinylphenyl)hydantoin.

7. A method for the manufacture of a hydrophilic microporous membrane according to claims 1-6, **characterized in that** a membrane for water sterilisation or for wound dressing is manufactured by thoroughly mixing an organic solvent, a hydrophilic substance, a substance that does or does not form pores, a hydrophilic polymer and a water insoluble biocidal polymer with the formula 1 or 2, wherein X, R, R' and n have the meanings defined in claim 3, after which the mixture is cast onto a support, coagulated in a water batch, optionally followed by treatment with an anorganic acid, rinsing and drying.

8. A method for the manufacture of an air filtration microporous membrane according to claims 1-6,
**characterized in that** a hydrophobic membrane is manufactured by thoroughly mixing a solvent, polysulphone, a pore-forming compound and a poly-1,3-dichloro(dibromo)-5-methyl-5-(4'-vinylphenyl)-hydantoin, after which the obtained mixture is cast onto a support forming a film, which is then washed, after which the pore-forming medium is dissolved in an acid, followed by rinsing and drying.

## Revendications

1. Membrane plane ou capillaire ou tubulaire microporeuse, contenant un composé biocide insoluble dans l'eau, **caractérisé en ce que** la matrice de la membrane contient comme composé biocide, un polymère de N-haloamine et dans laquelle la membrane contient de 0,5 à 20% en poids de composé biocide.

2. Membrane selon la revendication 1, **caractérisé en ce que** le polymère de N-haloamine est un polystyrène hydantoïne ayant la formule 1 : 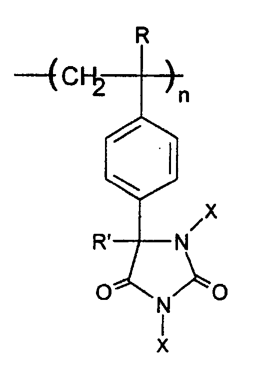 dans laquelle
X = Cl, Br ou I
R = H, CH₃
R' est un groupe alkyle
n = 10 - 50x10³
ou un polystyrène triazinedione ayant la formule 2: 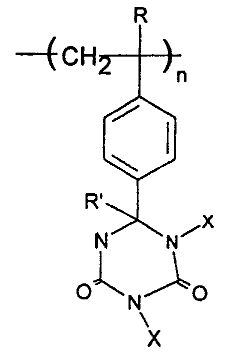 dans laquelle
X= Cl, Br ou I
R= H ou CH₃
R' est un groupe alkyle
n= 10 - 50x 10³

3. Membrane selon les revendications 1 ou 2, **caractérisée en ce que** la membrane contient en tant que composé biocide, de la poly 1,3-dichloro (dibromo ou di-iodo)-5-methyl 5- (4'-vinylphenyl)hydantoïne.

4. Membrane selon la revendication 3, convenant pour la stérilisation de l'eau **caractérisée en ce que** la membrane contient de 0,5 à 12% en poids de poly 1,3-dichloro(dibromo)-5-methyl-5-(4'-vinylphenyl)hydantoïne.

5. Membrane selon la revendication 3 convenant pour la filtration de l'air, **caractérisée en ce que** la membrane contient de 12 à 25% en poids de poly-1,3-dichloro(dibromo)-5-methyl-5-(4'-vinylphenyl)hydantoïne.

6. Membrane selon la revendication 3, convenant pour pansement des plaies stériles **caractérisée en ce que** la membrane contient de 0,5 à 12% en poids de poly-1,3-dichloro(dibromo)-5-methyl-5-(4'-vinylphenyl)hydantoïne

7. Procédé de fabrication d'une membrane microporeuse hydrophile selon les revendications là 6, **caractérisé en ce qu'**une membrane destinée à la stérilisation de l'eau ou pour le pansement des plaies est fabriquée en mélangeant soigneusement un solvant organique, une substance hydrophile, une substance qui doit ou qui ne doit pas former de pores, un polymère hydrophile et un polymère biocide insoluble dans l'eau ayant la formule 1 ou la formule 2, dans laquelle X, R, R' et n ont les significations définies à la revendication 3, après quoi le mélange est projeté sur un support, coagulé dans un bain d'eau, éventuellement suivi par un traitement par un acide minéral, rinçage et séchage.

8. Procédé pour la fabrication d'une membrane microporeuse destinée à la filtration de l'air selon les revendications 1 à 6, **caractérisé en ce qu'**une membrane hydrophobe est fabriquée en mélangeant soigneusement un solvant, une polysulfone, un composé porogène et une poly-1,3-dichloro(dibromo)-5-methyl-5-(4'-vinylphenyl)-hydantoïne, après quoi le mélange obtenu est projeté sur un support en formant une pellicule, qui est alors lavée, après quoi l'agent porogène est dissout dans un acide, suivi par un rinçage et un séchage.

## Patentansprüche

1. Eine mikroporöse Flach- oder Kapillar- oder Rohrmembran, die eine wasserunlösliche Biozidverbindung umfasst, **dadurch gekennzeichnet, dass** die Matrix der Membran als Biozidverbindung ein N-Halaminpolymer umfasst und wobei die Membran 0,5 - 25 Gew.% der Biozidverbindung umfasst.

2. Eine Membran nach Anspruch 1, **dadurch gekennzeichnet, dass** das N-Halaminpolymer ein Polystyrenhydantoin mit der Formel 1 ist 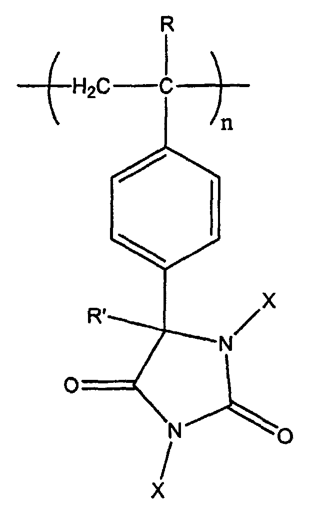 wobei
X = Cl, Br, J
R = H, CH₃
R'= eine Alkylgruppe
n = 10-50.10³
oder ein Polystyrentriazindion mit der Formel 2 ist 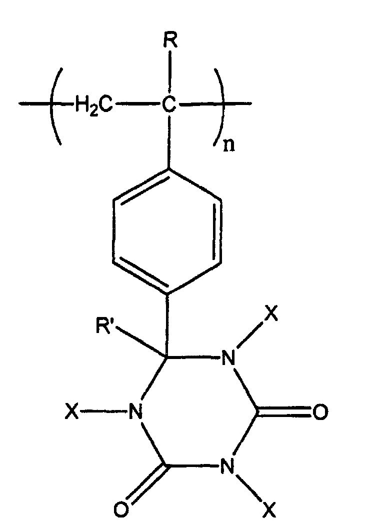 wobei
X = Cl, Br, J
R = H, CH₃
R'= eine Alkylgruppe
n = 10-50.10³

3. Eine Membran nach den Ansprüchen 1 oder 2, **dadurch gekennzeichnet, dass** die Membran als Biozidverbindung Poly-1,3-dichloro(dibromo, di-iodo)-5-methyl-5-(4'vinylphenyl)hydantoin umfasst.

4. Eine Membran nach Anspruch 3, geeignet für die Wassersterilisierung, **dadurch gekennzeichnet, dass** die Membran 0,5 - 12 Gew.% an Poly-1,3-dichloro(dibromo)-5-methyl-5-(4'-vinylphenyl)hydantoin umfasst.

5. Eine Membran nach Anspruch 3, geeignet für die Luftfiltration, **dadurch gekennzeichnet, dass** die Membran 12 - 25 Gew.% an Poly-1,3-dichloro(dibromo)-5-methyl-5-(4'vinylphenyl)hydantoin umfasst.

6. Eine Membran nach Anspruch 3, geeignet für sterile Wundabdeckung, **dadurch gekennzeichnet, dass** die Membran 0,5 -12 Gew.% an Poly-1,3-di-iodo-5-methyl-5-(4'vinylphenyl)hydantoin umfasst.

7. Ein Verfahren zur Herstellung einer hydrophilen mikroporösen Membran nach den Ansprüchen 1 - 6, **dadurch gekennzeichnet, dass** eine Membran zur Wassersterilisierung oder zur Wundabdeckung hergestellt wird durch gründliches Vermischen eines organischen Lösungsmittels, einer hydrophilen Substanz, einer Substanz, welche Poren ausbildet oder keine Poren ausbildet, eines hydrophilen Polymers und eines wasserunlöslichen Biozidpolymers mit der Formel 1 oder 2, wobei X, R, R' und n die in Anspruch 3 definierten Bedeutungen haben, nach welchem das Gemisch auf einen Träger gegossen wird, in einem Wasser-Batch koaguliert wird, gegebenenfalls gefolgt von einer Behandlung mit einer anorganischen Säure, Abspülen und Trocknen.

8. Ein Verfahren zur Herstellung einer mikroporösen Membran zur Luftfiltration nach den Ansprüchen 1 - 6, **dadurch gekennzeichnet, dass** eine hydrophobe Membran hergestellt wird durch gründliches Vermischen eines Lösungsmittels, Polysulfons, einer porenbildenden Verbindung und eines Poly-1,3-dichloro(dibromo)-5-methyl-5-(4'-vinylphenyl)-hydantoin, nach welchem das erhaltene Gemisch auf einen Träger unter Ausbildung eines Film gegossen wird, welcher anschließend gewaschen wird, nach welchem das porenbildende Medium in einer Säure aufgelöst wird, gefolgt durch Abspülen und Trocknen.
